# EUROPEAN PATENT APPLICATION

(11) **EP 4 671 768 A1**
(43) Date of publication of application: **31.12.2025**
(21) Application number: 23923659.9
(22) Date of filing: 06.09.2023
(51) Int. Cl.: G01N 33/72, G01N 33/52, G01N 21/17

(54) **REAGENT, KIT AND TEST METHOD FOR TESTING GLYCOSYLATED HEMOGLOBIN**

(30) Priority: 21.02.2023 CN 202310140999
(71) Applicant: Acon Biotech (Hangzhou) Co., Ltd., Hangzhou, Zhejiang 310030 (CN)
(72) Inventor: QIN, Yan, Hangzhou, Zhejiang 310030 (CN); GAO, Kexiang, Hangzhou, Zhejiang 310030 (CN); SUN, Yulong, Hangzhou, Zhejiang 310030 (CN); ZHANG, Li, Hangzhou, Zhejiang 310030 (CN)
(74) Representative: Schiweck Weinzierl Koch Patentanwälte Partnerschaft mbB
(86) International application number: PCT/CN2023/117291
(87) International publication number: WO 2024/174492

(57) **Abstract**

The present invention provides a kit and a test method for testing glycated hemoglobin. The kit includes a test card, a reagent R1, and a reagent R2, where the test card includes a filtration membrane, the reagent R1 includes a buffer system, a surfactant, and an active reactant, and the reagent R2 includes a buffer system and a surfactant. In the present invention, by combining different types of surfactants in a certain proportion, a reagent for determining glycated hemoglobin has good thermal stability and can be transported and preserved at normal temperature.

## Description

### TECHNICAL FIELD

The present invention relates to the field of in vitro diagnostics, specifically to a reagent, a kit, and a test method for determining glycated hemoglobin.

### BACKGROUND

Diabetes has become the world's leading chronic disease. In China, due to its large population and being in a critical period of economic transformation, the incidence of diabetes is also showing a rapid upward trend. The treatment and detection of diabetes are inseparable. With the gradual understanding of diabetes knowledge, most people have realized the importance of monitoring fasting blood glucose and 2-hour postprandial blood glucose, and often take the measured values of the two as the standards for blood glucose control. However, this is not the case. Fasting blood glucose and 2-hour postprandial blood glucose are the standards for diagnosing diabetes, while the standard for measuring the level of diabetes control is glycated hemoglobin. Fasting blood glucose and postprandial blood glucose reflect the blood glucose level at a specific time and are easily affected by related factors such as food intake and glucose metabolism. Glycated hemoglobin can stably and reliably reflect the average blood glucose level within 2-3 months before the test, and is not significantly interfered by factors such as blood collection time, fasting status, and insulin use. Therefore, the Asia-Pacific Guidelines for the Prevention and Treatment of Diabetes issued by the International Diabetes Federation clearly stipulate that glycated hemoglobin is the internationally recognized "gold standard" for diabetes monitoring.

The detection methods for glycated hemoglobin mainly include affinity chromatography, chromatography, immunoassays, chemiluminescence, enzymatic methods, isoelectric focusing, electrophoresis, etc. Affinity chromatography is characterized by no interference from variants, fast reaction time, and convenient testing.

The principle of boronic acid affinity chromatography is that boronic acid active substances directionally label glycated hemoglobin in blood samples. Through microporous retention technology, glycated hemoglobin and total hemoglobin in blood are retained and detected separately at specific wavelengths. The signals are converted into glycated hemoglobin results and displayed on the screen of the tester. It is characterized by convenient testing, a small footprint, and accurate results. However, reagents for detecting glycated hemoglobin by such methods usually require refrigerated conditions for transportation and storage, and have a short shelf life. These transportation conditions are too stringent: during transportation, the temperature must be maintained within the range of 2-8°C, requiring real-time monitoring, which leads to high transportation costs and difficulties in supervision. Cold chain transportation requires finding qualified agents, but there are relatively few agents with cold chain transportation qualifications, which limits the market promotion and sales volume of products, and to a certain extent, greatly restricts market supply.

### SUMMARY

In view of the problem of stringent preservation conditions in the prior art, the present invention optimizes the formulation. Through continuous research, it has been found that the combined use of different types of surfactants can significantly improve the high-temperature tolerance of the formulation, greatly reduce transportation costs, facilitate better promotion and expansion of product sales in various regions, and simultaneously improve the convenience of user experience. Therefore, the present invention provides a kit for determining glycated hemoglobin, including: a filtration membrane, a reagent R1, and a reagent R2, where the filtration membrane is capable of retaining precipitated hemoglobin, the reagent R1 includes a boronic acid conjugate, a zinc ion, and a surfactant, and the reagent R2 includes a buffer solution and a surfactant; where the surfactant of the reagent R1 includes a nonionic surfactant and an anionic surfactant.

Further, in the reagent R1, the nonionic surfactant is selected from Triton X-100, and the anionic surfactant is selected from SDS.

Further, in the reagent R1, a content of Triton X-100 is 0.01%-0.5% (w/w), and a content of SDS is 0.01%-0.5% (w/w).

In a preferred embodiment, in the reagent R1, the content of Triton X-100 is 0.05%-0.5% (w/w), and the content of SDS is 0.01%-0.1% (w/w).

Further, the surfactant in the reagent R1 includes a surfactant capable of lysing an erythrocyte.

Further, the surfactant in the reagent R1 further includes a zwitterionic surfactant.

Further, the zwitterionic surfactant in the reagent R1 is selected from one or both of Tetronic 1307 and CHAPS.

Further, a content of the zwitterionic surfactant in the reagent R1 is 0.01%-0.5% (w/w).

In a preferred embodiment, the content of the zwitterionic surfactant in the reagent R1 is 0.01%-0.1% (w/w).

Further, a content of the boronic acid conjugate is 0.01%-0.06% (w/w).

Further, the filtration membrane is disposed within a test card, the test card includes a sample loading port, and a sample to be tested and a reagent reach the filtration membrane through the sample loading port.

The present invention provides a method for determining glycated hemoglobin, including:
mixing a blood sample with a reagent R1 to form a mixed sample;
passing the mixed sample through a filtration membrane capable of retaining precipitated hemoglobin;
rinsing a filtration membrane retaining the precipitated hemoglobin with a reagent R2; and
separately detecting amounts of the glycated hemoglobin and total hemoglobin, and in turn calculating a percentage of the glycated hemoglobin,
where
the reagent R1 includes a boronic acid conjugate, a zinc ion, and a surfactant; and
the reagent R2 includes a buffer solution and a surfactant,
where the surfactant in the reagent R1 includes a nonionic surfactant and an anionic surfactant.

Further, in the reagent R1, the nonionic surfactant is selected from Triton X-100, and the anionic surfactant is selected from SDS.

Further, in the reagent R1, a content of Triton X-100 is 0.01%-0.5% (w/w), and a content of SDS is 0.01%-0.5% (w/w).

In a preferred embodiment, in the reagent R1, the content of Triton X-100 is 0.05%-0.5% (w/w), and the content of SDS is 0.01%-0.1% (w/w).

Further, the surfactant in the reagent R1 includes a surfactant capable of lysing an erythrocyte.

Further, the surfactant in the reagent R1 further includes a zwitterionic surfactant.

Further, the zwitterionic surfactant in the reagent R1 is selected from one or both of Tetronic 1307 and CHAPS.

Further, a content of the zwitterionic surfactant in the reagent R1 is 0.01%-0.5% (w/w).

In a preferred embodiment, the content of the zwitterionic surfactant in the reagent R1 is 0.01%-0.1% (w/w).

Further, a content of the boronic acid conjugate is 0.01%-0.06% (w/w).

The present invention further provides a reagent for determining glycated hemoglobin, including a reagent R1 and a reagent R2, where the reagent R1 includes an active reactant, a metal ion, and a surfactant, and the reagent R2 is an eluent, where the surfactant in the reagent R1 includes a nonionic surfactant and an anionic surfactant.

Further, in the reagent R1, the nonionic surfactant is selected from Triton X-100, and the anionic surfactant is selected from SDS.

Further, in the reagent R1, a content of Triton X-100 is 0.01%-0.5% (w/w), and a content of SDS is 0.01%-0.5% (w/w).

In a preferred embodiment, in the reagent R1, the content of Triton X-100 is 0.05%-0.5% (w/w), and the content of SDS is 0.01%-0.1% (w/w).

Further, the surfactant in the reagent R1 includes a surfactant capable of lysing an erythrocyte.

Further, the surfactant in the reagent R1 further includes a zwitterionic surfactant.

Further, the zwitterionic surfactant in the reagent R1 is selected from one or both of Tetronic 1307 and CHAPS.

Further, a content of the zwitterionic surfactant in the reagent R1 is 0.01%-0.5% (w/w).

In a preferred embodiment, the content of the zwitterionic surfactant in the reagent R1 is 0.01%-0.1% (w/w).

Further, the active reactant is a boronic acid conjugate.

Further, a content of the boronic acid conjugate is 0.01%-0.06% (w/w).

Further, the metal ion in the reagent R1 includes a zinc ion.

Further, the metal ion in the reagent R1 further includes a sodium ion and a magnesium ion.

Further, the reagent R2 includes a buffer solution and a surfactant.

The present invention further provides a method for preserving a reagent, where the reagent includes a boronic acid conjugate, a zinc ion, and a surfactant, where the surfactant includes a nonionic surfactant and an anionic surfactant.

Further, in the reagent R1, the nonionic surfactant is selected from Triton X-100, and the anionic surfactant is selected from SDS.

Further, in the reagent R1, a content of Triton X-100 is 0.01%-0.5% (w/w), and a content of SDS is 0.01%-0.5% (w/w).

In a preferred embodiment, in the reagent R1, the content of Triton X-100 is 0.05%-0.5% (w/w), and the content of SDS is 0.01%-0.1% (w/w).

Further, the surfactant in the reagent R1 further includes a zwitterionic surfactant.

Further, the zwitterionic surfactant in the reagent R1 is selected from one or both of Tetronic 1307 and CHAPS.

Further, a content of the zwitterionic surfactant in the reagent R1 is 0.01%-0.5% (w/w).

In a preferred embodiment, the content of the zwitterionic surfactant in the reagent R1 is 0.01%-0.1% (w/w).

Further, a content of the boronic acid conjugate is 0.01%-0.06% (w/w).

The beneficial effects of the present invention are as follows: in the present invention, through combined use of different types of surfactants in certain proportions, the thermal stability of the reagent for testing glycated hemoglobin (boronic acid affinity chromatography) is effectively improved, and transportation and storage conditions are improved and extended simultaneously. In the past, test cards for glycated hemoglobin based on boronic acid affinity chromatography usually required cold chain transportation and refrigerated storage. After formulation optimization and stability test verification of the present invention, the synergistic effect between surfactants can promote the overall improvement of the thermal stability of the formulation system. Under such conditions, the shelf life of the test cards for boronic acid affinity chromatography is extended to 6 months under normal temperature and reaches 18 months under refrigeration, with the bias between their performance test results and those of standard instruments being within an acceptable range.

### BRIEF DESCRIPTION OF THE DRAWINGS

FIG. 1 is a linear relationship between HbAlc test values and test values of a standard instrument G8 in Example 5.
FIG. 2 is a linear relationship between HbAlc test values of a kit after 6 months of storage at normal temperature and test values of a standard instrument G8 in Example 5.

### DETAILED DESCRIPTION OF THE EMBODIMENTS

The present invention is described in detail below in conjunction with specific examples.

The reagent for testing glycated hemoglobin of the present invention includes a reagent R1 and a reagent R2. The kit for testing glycated hemoglobin of the present invention includes a test card for the glycated hemoglobin, a reagent R1, and a reagent R2.

The test card for the glycated hemoglobin includes a filtration membrane and a plastic plate. The filtration membrane may be any membrane with a suitable pore size, such as a glass fiber membrane, a borosilicate membrane, or a cellulose membrane. The pore size of the membrane is 0.5-1.5 µm, and preferably 0.8-1.0 µm. The filtration membrane is mounted in the plastic plate, and the plastic plate is provided with a sample loading port. A sample to be tested and a test reagent reach the filtration membrane through the sample loading port.

The reagent R1 includes a buffer system and an active reactant. The reagent R2 includes a buffer system and a surfactant.

In a preferred embodiment, the buffer system of the reagent R1 includes a buffer solution, a metal ion, a surfactant, and an organic solvent.

The buffer solution is selected from one of HEPES and ammonium acetate.

The metal ion is selected from a zinc ion, and may further include a sodium ion and a magnesium ion.

The surfactant in the reagent R1 is selected from one or more types of surfactants of a nonionic surfactant, an anionic surfactant, and a zwitterionic surfactant. Preferably, the reagent R1 includes a dual surfactant system of the nonionic surfactant and the anionic surfactant, and more preferably, the reagent R1 includes a triple surfactant system of the nonionic surfactant, the anionic surfactant, and the zwitterionic surfactant. In the reagent R1, the nonionic surfactant is selected from Triton or Tween or a combination thereof, and preferably Traiton X-100 (polyethylene glycol octylphenyl ether); the anionic surfactant is selected from SDS (sodium dodecyl sulfate) or GEROPON T-77 (sodium N-oleoyl-N-methyltaurate) or a combination thereof, and preferably SDS; and the zwitterionic surfactant is selected from Tetronic 1307 (ethylenediamine alkoxy ester block copolymer), CHAPS (3-[3-(cholamidopropyl)dimethylammonio]propanesulfonate inner salt), or a combination thereof. Furthermore, the dual surfactant system of the reagent R1 includes Triton X-100 and SDS; or the triple surfactant system of the reagent R1 includes Triton X-100, SDS, and Tetronic 1307. In the dual surfactant system, Triton X-100 accounts for a content of about 0.01%-0.5% (w/w) in a final solution, and SDS accounts for a content of 0.01%-0.5% (w/w); and preferably, the content of Triton X-100 is 0.05%-0.5% (w/w), and the content of SDS is 0.01%-0.1% (w/w). In the triple surfactant system, Triton X-100 accounts for a content of about 0.01%-0.5% (w/w) in a final solution, SDS accounts for a content of 0.01%-0.5% (w/w), and Tetronic 1307 accounts for a content of 0.01%-0.5% (w/w); and preferably, the content of Triton X-100 is 0.05%-0.5% (w/w), the content of SDS is 0.01%-0.1% (w/w), and the content of Tetronic 1307 is 0.01%-0.1% (w/w).

The active reactant of the reagent R1 is selected from a boronic acid conjugate, including a boronic acid conjugate at a concentration of 0.01%-0.06% (w/w). The boronic acid conjugate is a coupling product of a chromophore and boronic acid.

The organic solvent of the reagent R1 is selected from dimethyl sulfoxide.

In the reagent R2, the buffer system is selected from one of HEPES and ammonium acetate, and the surfactant includes a nonionic surfactant at a concentration of 0.25% (w/w).

### Test principle:

The glycated hemoglobin test of the present invention is based on the principle of chromatography. A blood sample is added to the reagent R1, where the boronic acid conjugate binds to the glycated hemoglobin, and the zinc ions precipitate hemoglobin. A certain amount of the mixture of the reagent R1 and the blood sample is added to the reaction well of the test card. All hemoglobin and the boronic acid conjugate bound or unbound to the glycated hemoglobin are retained on the filtration membrane of the test card. The reagent R2 is added onto the filtration membrane, and the unbound boronic acid conjugate is removed by the reagent R2. The amounts of the glycated hemoglobin and total hemoglobin are detected separately using a glycated hemoglobin analyzer, and in turn, the percentage of the glycated hemoglobin is calculated.

The specific reaction principle is as follows:
Boronic acid conjugate + glycated hemoglobin → glycated hemoglobin boronate ester

### Test steps:

5 microliters of whole blood sample is taken and added to 200 microliters of the reagent 1, shaken 5-10 times, stewing 3 minutes, and shaken again 5-10 times. 25 microliters of the mixture of the reagent R1 is taken and added to the reaction well of the test card, and after waiting for 15 seconds, 25 microliters of the reagent R2 is taken and added to the reaction well of the test card. After waiting for 1 minute, the data at 625 nm and 470 nm (625 nm and 470 nm correspond to the measurements of the glycated hemoglobin and hemoglobin, respectively) are read from the glycated hemoglobin analyzer, and the percentage of the glycated hemoglobin is obtained through calculation.

Example 1 The surfactant in the formulation of the reagent R1 included only a nonionic surfactant

In this example, the reagent R1 uses a single surfactant system, mainly a nonionic surfactant that can be used to lyse samples, such as Triton X-100 (polyethylene glycol octylphenyl ether) and NP-40 (ethyl phenyl polyethylene glycol). The specific reagent formulations are as follows.

The reagent R1 includes a 0.2 M HEPES buffer solution, 0.2% (w/w) zinc ions, 0.1% (w/w) magnesium ions, 1.7% (w/w) sodium ions, 0.2% (w/w) Triton X-100, 20% (w/w) DMSO (dimethyl sulfoxide), and a 0.04% (w/w) boronic acid conjugate, and is prepared by mixing the above components, with the pH value adjusted to 8.10.

The reagent R2 includes a 0.2 M HEPES buffer solution and 0.25% (w/w) Triton X-100, and is prepared by mixing the above components, with a pH value of 8.50.

The test card for glycated hemoglobin includes an upper cover and a bottom plate, with glass fibers (manufacturer: Pall, model: A/B or A/D) mounted between the upper cover and the bottom plate. A sample loading port is formed on the upper cover, and reagents and samples to be tested reached the filtration membrane through the sample loading port.

Glycated hemoglobin analyzer: On·Call glycated hemoglobin analyzer.

### Test steps:

5 microliters of whole blood sample is added to 200 microliters of the reagent 1, shaken 5-10 times, stewing 3 minutes, and shaken again 5-10 times. 25 microliters of the mixture of the reagent 1 is added to the reaction well of the test card, and after waiting for 15 seconds, 25 microliters of the reagent 2 is added to the reaction well of the test card. After waiting for 1 minute, the test results at 625 nm and 470 nm are read from the glycated hemoglobin analyzer.

The reagent R1, the reagent R2, and the test card are preserved under 2-8°C conditions, and taken out at the 1st, 6th, 12th, and 15th months of preservation. They are rewarmed at 15-30°C for 15 minutes to test the glycated hemoglobin content in the whole blood sample. The test values of the standard instrument Tosoh G8 automated glycated hemoglobin analyzer (hereinafter referred to as standard instrument G8 or control G8 or G8; manufacturer: Tosoh Corporation, Japan) are used as controls. The test data are shown in Table 1.

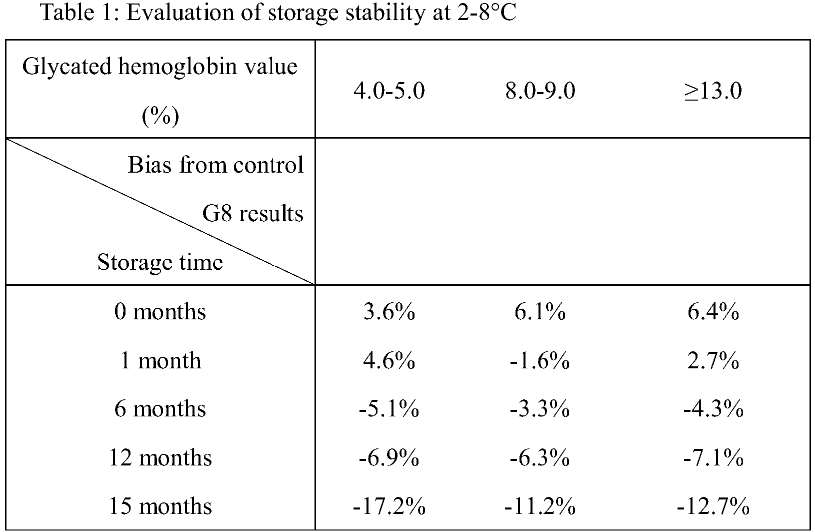

From the above data, it can be found that when under 2-8°C conditions, within 12 months, the biases of the test results from those of the standard instrument Tosoh G8 are all within the acceptable range. After preservation exceeds 12 months, the test results of some samples at specific concentrations show biases exceeding ±15%, indicating that when the reagent is preserved at 2-8°C for more than 12 months, its test results are beyond the acceptable range.

The reagent R1, the reagent R2, and the test card are preserved at normal temperature, and in the 1st and 2nd months, they are used to test the glycated hemoglobin content in the whole blood sample. The test values of the standard instrument Tosoh G8 automated glycated hemoglobin analyzer (manufacturer: Tosoh Corporation, Japan) are used as reference controls. The test results are shown in Table 2.

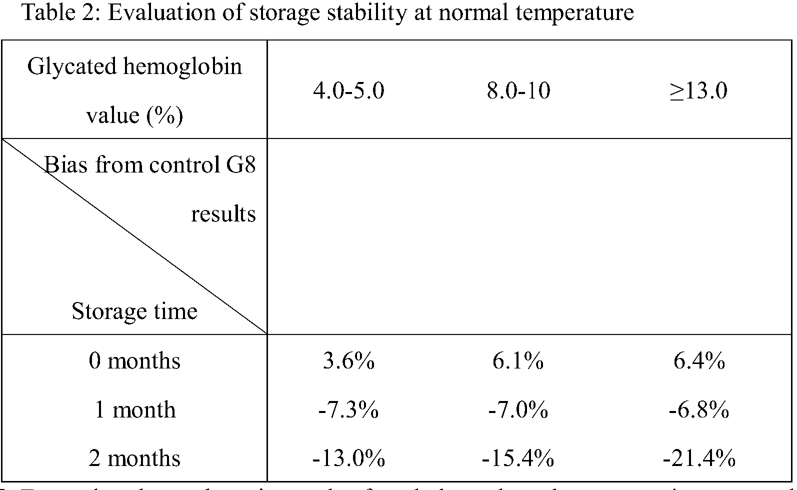

From the above data, it can be found that when the reagent is preserved at normal temperature for 1 month, the test results of glycated samples at different concentrations, when compared with the test values of the standard instrument G8, show all biases within the acceptable range. However, after preservation exceeds one month, the test results of some samples at specific concentrations exceed +15%, indicating that this system should not be preserved at normal temperature for more than 1 month.

The reagent R1, the reagent R2, and the test card are preserved under 37°C conditions. According to the Arrhenius equation, storage at 37°C for 8 days is equivalent to storage at 4°C for one year; and storage at 37°C for 16 days is equivalent to storage at 4°C for two years. The test values of the standard instrument Tosoh G8 automated glycated hemoglobin analyzer (manufacturer: Tosoh Corporation, Japan) are used as controls. The results are shown in Table 3.

**Table 3: Accelerated stability evaluation**

| G8 HbAlc value (%) | 4.4 | | | 8.6 | | | 14.6 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Reading (%) | 4.7 | 4.5 | 4.0 | 8.4 | 7.9 | 6.9 | 14.8 | 14.3 | 12.3 |
| | 4.8 | 4.7 | 4.1 | 8.5 | 7.9 | 7.1 | 14.3 | 13.5 | 11.5 |
| | 4.9 | 4.7 | 3.9 | 9.0 | 8.0 | 6.8 | 14.7 | 14.4 | 11.9 |
| | 4.6 | 4.6 | 4.1 | 8.8 | 8.0 | 6.9 | 14.2 | 14.4 | 12.0 |
| | 4.7 | 4.5 | 4.0 | 8.5 | 8.1 | 7.0 | 14.1 | 14.3 | 11.7 |
| Avg | 4.7 | 4.6 | 4.0 | 8.6 | 8.0 | 6.9 | 14.4 | 14.2 | 11.9 |
| SD | 0.12 | 0.11 | 0.08 | 0.25 | 0.11 | 0.11 | 0.32 | 0.36 | 0.30 |
| CV | 2.5% | 2.5% | 2.1% | 2.9% | 1.4% | 1.6% | 2.2% | 2.5% | 2.6% |
| %Bias vs G8 | 7.8% | 4.5% | -8.6% | 0.3% | -7.3% | -19.3% | -1.2% | -2.8% | -18.6% |
| %Bias vs 4°C | | -3.1% | -15.3% | | -7.6% | -19.6% | | -1.6% | -17.6% |

From the above data, it can be found that when the reagent is stored under 37°C conditions for 8 days (according to the Arrhenius equation, storage under 37°C conditions for 8 days is equivalent to preservation under refrigerated conditions for one year), its performance remains within the acceptable range. When the reagent is stored at 37°C for 16 days, which is equivalent to preservation under refrigerated conditions for 2 years, the bias between the test results and those of the standard instrument exceeds ±15%, indicating that when the reagent is stored for more than one year, the test results of some samples at specific concentrations show relatively large biases.

Accelerated stability experiments are conducted at 37°C, and the bias from the results of the reagent stored at 4°C is calculated using the ratio k/s value. The experimental results (see the table below 3-1) show that when the reagent is stored at 37°C for 16 days, the bias between its test results and those stored at 4°C exceeds ± 15%, indicating that when the reagent is stored for more than one year, the test results have relatively large biases.

**Table 3-1: Accelerated stability (ratio k/s)**

| G8 HbAlc value (%) | 4.6 | | | 9.0 | | | 12.8 | | |
|---|---|---|---|---|---|---|---|---|---|
| Treatment condition | 4°C | 37°C8 D | 37°C 16D | 4°C | 37°C8 D | 37°C 16D | 4°C | 37°C8 D | 37°C 16D |
| Ratio k/s | 0.177 | 0.158 | 0.135 | 0.446 | 0.394 | 0.347 | 0.608 | 0.587 | 0.501 |
| | 0.164 | 0.152 | 0.147 | 0.438 | 0.404 | 0.375 | 0.593 | 0.556 | 0.480 |
| | 0.171 | 0.153 | 0.143 | 0.460 | 0.414 | 0.359 | 0.639 | 0.525 | 0.503 |
| Avg | 0.171 | 0.154 | 0.142 | 0.448 | 0.404 | 0.360 | 0.613 | 0.556 | 0.495 |
| SD | 0.007 | 0.003 | 0.006 | 0.011 | 0.010 | 0.014 | 0.023 | 0.031 | 0.013 |
| CV | 3.8% | 2.1% | 4.3% | 2.5% | 2.5% | 3.9% | 3.8% | 5.6% | 2.6% |
| %Bias vs 4°C | | -9.6% | -17.0% | | -9.8% | -19.6% | | -9.3% | -19.3% |

Example 2 The formulation of the reagent R1 includes a nonionic surfactant and an anionic surfactant

In this example, a dual surfactant system, namely a nonionic surfactant and an anionic surfactant, is adopted in the formulation of the reagent R1. The specific reagent formulations are as follows.

The reagent R1 includes a 0.2 M HEPES buffer solution, 0.2% (w/w) zinc ions, 0.1% (w/w) magnesium ions, 1.7% (w/w) sodium ions, 0.1% (w/w) Triton X-100, 20% (w/w) DMSO (dimethyl sulfoxide), 0.04% (w/w) boronic acid conjugate, and 0.05% (w/w) SDS (sodium dodecyl sulfate), and is prepared by mixing the above components, with a pH value of 8.10.

The reagent R2 and the test card are the same as those in Example 1.

The reagent R1, the reagent R2, and the test card are preserved under 37°C conditions. According to the Arrhenius equation, storage at 37°C for 8 days is equivalent to storage at 4°C for one year; and storage at 37°C for 16 days is equivalent to storage at 4°C for two years. The test results of the kit stored at 4°C are used as reference controls. The results are shown in Table 4.

**Table 4 Accelerated stability evaluation**

| G8 HbAlc value (%) | 4.7 | | | 8.3 | | | 13.1 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Ratio k/s | 0.177 | 0.163 | 0.171 | 0.375 | 0.328 | 0.329 | 0.688 | 0.683 | 0.617 |
| | 0.188 | 0.165 | 0.154 | 0.358 | 0.341 | 0.293 | 0.696 | 0.616 | 0.579 |
| | 0.182 | 0.172 | 0.167 | 0.358 | 0.317 | 0.319 | 0.654 | 0.639 | 0.598 |
| Avg | 0.182 | 0.167 | 0.164 | 0.363 | 0.329 | 0.314 | 0.679 | 0.646 | 0.598 |
| SD | 0.01 | 0.00 | 0.01 | 0.01 | 0.01 | 0.02 | 0.02 | 0.03 | 0.02 |
| CV | 2.8% | 2.9% | 5.4% | 2.7% | 3.6% | 5.8% | 3.3% | 5.3% | 3.2% |
| %Bias vs 4°C | | -8.5% | -10.1% | | -9.6% | -13.7% | | -4.8% | -12.0% |

According to the Kubelka-Munk theory, there is a certain correlation between the ratio of the k/s value of glycated hemoglobin (K: absorption coefficient in solids, and S: scattering coefficient) at different concentrations to the k/s value of total hemoglobin and the glycated hemoglobin concentration value (%). The bias between this ratio and the results at 4°C can well reflect the bias between the test results under different temperatures and durations and those of the test card preserved at 4°C. Therefore, the data in this study are compared using the ratio of the k/s value of glycated hemoglobin to the k/s value of total hemoglobin. It is found that when the kit is stored under different temperatures and durations, the bias between the test results of samples with different concentrations and those stored at 4°C does not exceed ±15%, indicating that the bias between the final test results and the control is within the range of ±15%.

### Example 3

In this example, the content of SDS in the formulation of the reagent R1 is 0.01 % (w/w). The other components and concentrations of the reagent R1, as well as the reagent R2 and the test card, are the same as those in Example 2.

The reagent R1, the reagent R2, and the test card are preserved under 37°C conditions. According to the Arrhenius equation, storage at 37°C for 8 days is equivalent to storage at 4°C for one year; and storage at 37°C for 16 days is equivalent to storage at 4°C for two years. The test results of the kit stored at 4°C are used as reference controls. The results are shown in Table 5.

**Table 5: Accelerated stability evaluation**

| G8 HbAlc value (%) | 4.5 | | | 8.8 | | | 14.7 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Ratio k/s | 0.184 | 0.167 | 0.158 | 0.363 | 0.325 | 0.316 | 0.663 | 0.592 | 0.576 |
| | 0.191 | 0.169 | 0.159 | 0.355 | 0.337 | 0.316 | 0.673 | 0.591 | 0.559 |
| | 0.181 | 0.160 | 0.160 | 0.374 | 0.311 | 0.301 | 0.656 | 0.575 | 0.566 |
| Avg | 0.185 | 0.165 | 0.159 | 0.364 | 0.324 | 0.311 | 0.664 | 0.586 | 0.567 |
| SD | 0.01 | 0.00 | 0.00 | 0.01 | 0.01 | 0.01 | 0.01 | 0.01 | 0.01 |
| CV | 2.8% | 3.0% | 0.6% | 2.6% | 3.9% | 2.7% | 1.3% | 1.6% | 1.5% |
| %Bias vs 4°C | | -10.8% | -14.2% | | -10.9% | -14.6% | | -11.7% | -14.6% |

The data in this study are compared using the ratio of the k/s value of glycated hemoglobin to the k/s value of total hemoglobin. It is found that when the kit is stored under different temperatures and durations, the bias between the test results of samples with different concentrations and those stored at 4°C does not exceed ±15%.

Example 4 The surfactant in the formulation of the reagent includes only an anionic surfactant

In this example, the reagent R1 includes a 0.2 M HEPES buffer solution, 0.2% (w/w) zinc ions, 0.1% (w/w) magnesium ions, 1.7% (w/w) sodium ions, 0.1% (w/w) SDS (sodium dodecyl sulfate), 20% (w/w) DMSO (dimethyl sulfoxide), and 0.04% (w/w) boronic acid conjugate, and is prepared by mixing the above components, with a pH value of 8.10. The reagent R2 and the test card are the same as those in Example 1.

The reagent R1, the reagent 2, and the test card are tested at different concentrations according to the experimental steps. It is found that when the reagent R1 includes only the anionic surfactant SDS, the test results are unsatisfactory. There is no gradient between different concentrations, and the results are basically at the same level, indicating that using only SDS is not suitable for this system. The results are shown in Table 6.

**Table 6:**

| G8 HbAlc value (%) | 4.9 | 8.6 | 12.2 |
|---|---|---|---|
| Ratio k/s | 0.754 | 0.803 | 0.855 |
| | 0.785 | 0.787 | 0.853 |
| | 0.792 | 0.788 | 0.948 |
| Avg | 0.777 | 0.793 | 0.885 |
| SD | 0.02 | 0.01 | 0.05 |
| CV | 2.6% | 1.2% | 6.1% |

Example 5 The formulation of the reagent R1 includes a nonionic surfactant, an anionic surfactant, and a zwitterionic surfactant

In this example, three types of surfactants, namely a nonionic surfactant, an anionic surfactant, and a zwitterionic surfactant, are adopted in the formulation of the reagent R1. The zwitterionic surfactant includes Tetronic 1307, CHAPS, etc.

The specific reagent formulation of this example is as follows.

The reagent R1 includes a 0.2 M HEPES buffer solution, 0.2% (w/w) zinc ions, 0.1% (w/w) magnesium ions, 1.7% (w/w) sodium ions, 0.1% (w/w) Triton X-100, 20% (w/w) DMSO (dimethyl sulfoxide), 0.04% (w/w) boronic acid conjugate, 0.05% (w/w) SDS (sodium dodecyl sulfate), and 0.03% (w/w) Tetronic 1307, and is prepared by mixing the above components, with a pH value of 8.10.

The reagent R2 and the test card are the same as those in Example 1.

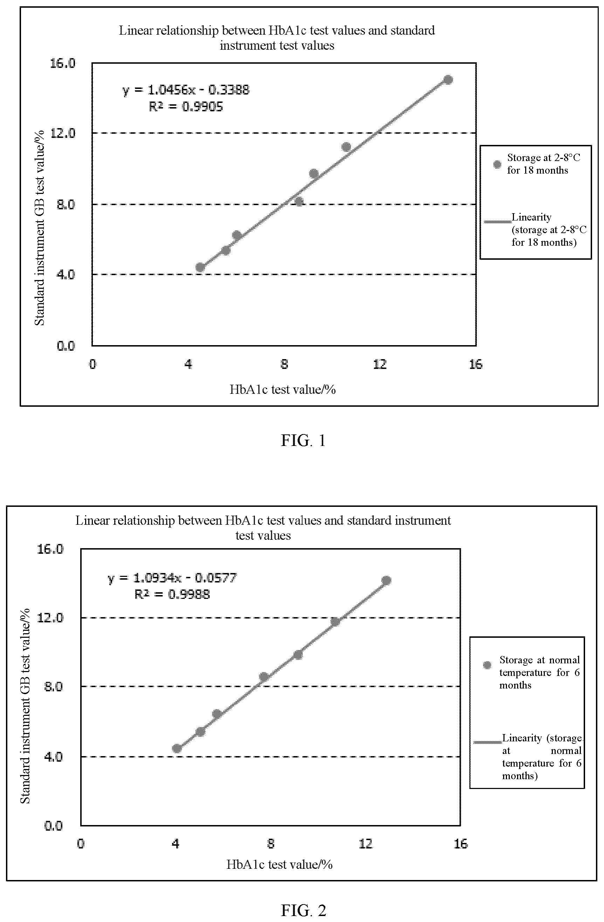

The reagent R1, the reagent 2, and the test card are preserved under 2-8°C conditions, and taken out at the 1st, 6th, 12th, and 15th months of preservation. They are rewarmed at 15-30°C for 15 minutes to test the glycated hemoglobin content in the whole blood sample. The test values of the standard instrument Tosoh G8 automated glycated hemoglobin analyzer (manufacturer: Tosoh Corporation, Japan) are used as controls. The test results are shown in Table 7 and FIG. 1.

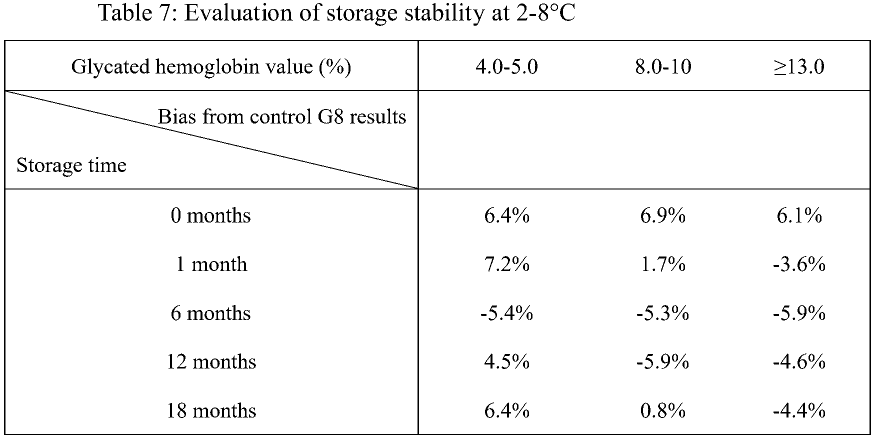

According to the data in Table 7 and FIG. 1, it can be found that when the kit is stored at 2-8°C, after 18 months, the bias of results from those of the G8 analyzer is within ±10% for samples at different concentrations, indicating relatively good test results. Additionally, the linear equation y = 1.0456x-0.3388 exhibits a slope between 0.90 and 1.10, and R2 ≥ 0.99, demonstrating good linear correlation and relatively high accuracy between the results and the test values of the standard instrument G8.

The reagent R1, the reagent 2, and the test card are preserved at normal temperature, and used to test the glycated hemoglobin content in the whole blood sample at the 1st, 3rd, and 6th months of preservation. The test values of the standard instrument Tosoh G8 automated glycated hemoglobin analyzer (manufacturer: Tosoh Corporation, Japan) are used as controls. The test results are shown in Table 8 and FIG. 2.

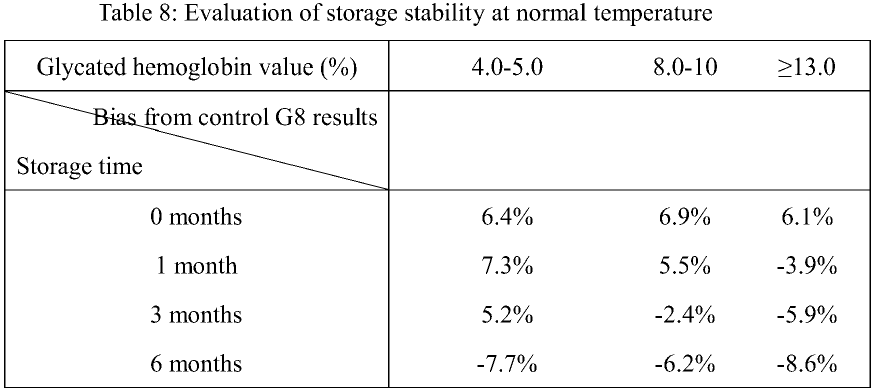

According to the data in Table 8 and FIG. 2, it can be found that when the kit is stored at normal temperature, after 6 months, the bias of results from those of the G8 analyzer is within ±10% for samples at different concentrations, indicating relatively good test results. Additionally, the linear equation y = 1.0934x-0.0577 exhibits a slope between 0.90 and 1.10, and R2 ≥ 0.99, demonstrating good linear correlation and relatively high accuracy between the results and the test values of the standard instrument G8.

The reagent R1, the reagent 2, and the test card are preserved at 37°C for 16 days, with the test values from the Tosoh G8 (manufacturer: Tosoh Corporation, Japan) automated glycated hemoglobin analyzer as controls. According to the Arrhenius law, storage at 37°C for 16 days is equivalent to storage at 4°C for two years. The test results are shown in Table 9.

**Table 9: Accelerated stability evaluation:**

| G8 HbAlc value (%) | 4.4 | | | 8.7 | | | 13.1 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Reading (%) | 4.7 | 4.8 | 4.7 | 9.6 | 8.9 | 8.3 | 13.0 | 12.8 | 12.0 |
| | 4.6 | 4.8 | 4.8 | 9.4 | 8.6 | 8.6 | 13.0 | 12.0 | 12.5 |
| | 4.8 | 4.8 | 4.6 | 9.2 | 9.3 | 8.7 | 13.6 | 12.8 | 12.1 |
| | 4.7 | 4.5 | 4.5 | 9.2 | 8.8 | 8.4 | 12.6 | 12.5 | 11.9 |
| | 4.9 | 4.7 | 4.8 | 8.9 | 9.1 | 8.7 | 13.1 | 12.5 | 11.9 |
| Avg | 4.7 | 4.7 | 4.7 | 9.3 | 9.0 | 8.5 | 13.0 | 12.5 | 12.1 |
| SD | 0.10 | 0.11 | 0.12 | 0.27 | 0.25 | 0.18 | 0.38 | 0.34 | 0.28 |
| CV | 2.1% | 2.3% | 2.6% | 2.9% | 2.8% | 2.1% | 2.9% | 2.7% | 2.3% |
| %Bias vs G8 % | 7.5% | 7.3% | 6.2% | 6.7% | 2.9% | -1.7% | -0.4% | -4.5% | -7.8% |
| Bias vs 4°C | | -0.2% | -1.2% | | -3.5% | -7.9% | | -4.1% | -7.4% |

According to the data in Table 9, it can be found that when the reagent is stored under 37°C conditions for 8 days (according to the Arrhenius equation, storage under 37°C conditions for 8 days is equivalent to preservation under refrigerated conditions for one year), its performance remains within the acceptable range; and when the reagent is stored under 37°C conditions for 16 days, the bias of results with samples with different concentrations compared with those of G8 is within ±10%, and the test results are relatively good.

**Table 10: Accelerated stability (ratio k/s)**

| G8 HbAlc value (%) | 4.4 | | | 8.3 | | | 14.5 | | |
|---|---|---|---|---|---|---|---|---|---|
| Treatment condition | 4°C | 37°C 8D | 37°C 16D | 4°C | 37°C 8D | 37°C 16D | 4°C | 37°C8D | 37°C16D |
| Ratio K/S | 0.218 | 0.214 | 0.201 | 0.453 | 0.428 | 0.412 | 0.817 | 0.775 | 0.782 |
| | 0.212 | 0.222 | 0.204 | 0.428 | 0.424 | 0.406 | 0.780 | 0.778 | 0.746 |
| | 0.219 | 0.208 | 0.199 | 0.444 | 0.417 | 0.417 | 0.813 | 0.779 | 0.745 |
| | 0.212 | 0.216 | 0.197 | 0.434 | 0.408 | 0.401 | 0.789 | 0.732 | 0.742 |
| Avg | 0.215 | 0.215 | 0.200 | 0.440 | 0.419 | 0.409 | 0.800 | 0.766 | 0.754 |
| SD | 0.00 | 0.01 | 0.00 | 0.01 | 0.01 | 0.01 | 0.02 | 0.02 | 0.02 |
| CV | 1.7% | 2.7% | 1.5% | 2.6% | 2.1% | 1.7% | 2.3% | 2.9% | 2.5% |
| %Bias vs 4°C | | -0.2% | -7.0% | | -4.6% | -6.9% | | -4.2% | -5.7% |

The data in Table 10 are compared using the ratio of the k/s value of glycated hemoglobin to the k/s value of total hemoglobin. It is found that when the kit is stored under different temperatures and durations, the bias between the test results of samples with different concentrations and those stored at 4°C does not exceed ± 15%. Moreover, the results show a smaller bias compared with the dual surfactant system.

### Example 6

In this example, the content of Tetronic 1307 in the formulation of the reagent R1 is 0.1% (w/w). The other components and concentrations of the reagent R1, as well as the reagent R2 and the test card, are the same as those in Example 5.

The reagent R1, the reagent 2, and the test card are preserved under 37°C conditions. According to the Arrhenius equation, storage at 37°C for 8 days is equivalent to storage at 4°C for one year; and storage at 37°C for 16 days is equivalent to storage at 4°C for two years. The test results of the kit stored at 4°C are used as reference controls. The results are shown in Table 11.

**Table 11: Accelerated stability evaluation:**

| G8 HbAlc value (%) | 4.9 | | | 8.3 | | | 14.2 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Ratio k/s | 0.212 | 0.198 | 0.194 | 0.406 | 0.357 | 0.355 | 0.711 | 0.607 | 0.616 |
| | 0.227 | 0.210 | 0.182 | 0.396 | 0.368 | 0.380 | 0.715 | 0.659 | 0.578 |
| | 0.215 | 0.199 | 0.197 | 0.380 | 0.342 | 0.354 | 0.659 | 0.636 | 0.591 |
| Avg | 0.218 | 0.203 | 0.191 | 0.394 | 0.356 | 0.363 | 0.695 | 0.634 | 0.595 |
| SD | 0.01 | 0.01 | 0.01 | 0.01 | 0.01 | 0.01 | 0.03 | 0.03 | 0.02 |
| CV | 3.4% | 3.2% | 4.1% | 3.3% | 3.7% | 3.9% | 4.5% | 4.1% | 3.3% |
| %Bias vs 4°C | | -7.1% | -12.3% | | -9.6% | -7.8% | | -8.8% | -14.4% |

The data in this study are compared using the ratio of the k/s value of glycated hemoglobin to the k/s value of total hemoglobin. It is found that when the kit is stored under different temperatures and durations, the bias between the test results of samples with different concentrations and those stored at 4°C does not exceed ± 15%.

### Example 7

In this example, the content of Tetronic 1307 in the formulation of the reagent R1 is 0.01% (w/w). The other components and concentrations of the reagent R1, as well as the reagent R2 and the test card, are the same as those in Example 5.

The reagent R1, the reagent 2, and the test card are preserved under 37°C conditions. According to the Arrhenius equation, storage at 37°C for 8 days is equivalent to storage at 4°C for one year; and storage at 37°C for 16 days is equivalent to storage at 4°C for two years. The test results of the kit stored at 4°C are used as reference controls. The results are shown in Table 12.

**Table 12: Accelerated stability evaluation:**

| G8 HbAlc value (%) | 4.2 | | | 7.4 | | | 12.9 | | |
|---|---|---|---|---|---|---|---|---|---|
| Treatment condition | 4°C | 37°C 8D | 37°C 16D | 4°C | 37°C 8D | 37°C 16D | 4°C | 37°C 8D | 37°C 16D |
| Ratio K/S | 0.143 | 0.143 | 0.129 | 0.275 | 0.256 | 0.256 | 0.634 | 0.571 | 0.541 |
| | 0.139 | 0.131 | 0.130 | 0.293 | 0.295 | 0.263 | 0.633 | 0.631 | 0.538 |
| | 0.160 | 0.149 | 0.121 | 0.283 | 0.252 | 0.242 | 0.614 | 0.588 | 0.527 |
| Avg | 0.147 | 0.141 | 0.127 | 0.284 | 0.268 | 0.254 | 0.627 | 0.597 | 0.535 |
| SD | 0.01 | 0.01 | 0.01 | 0.01 | 0.02 | 0.01 | 0.01 | 0.03 | 0.01 |
| CV | 7.7% | 6.5% | 4.0% | 3.2% | 8.9% | 4.0% | 1.8% | 5.2% | 1.4% |
| %Bias vs 4°C | | -4.1% | -14.0% | | -5.6% | -10.6% | | -4.8% | -14.6% |

The data in this study are compared using the ratio of the k/s value of glycated hemoglobin to the k/s value of total hemoglobin. It is found that when the kit is stored under different temperatures and durations, the bias between the test results of samples with different concentrations and those stored at 4°C does not exceed ±15%.

Example 8 The surfactant in the formulation of the reagent R1 includes only a zwitterionic surfactant

When only Tetronic 1307 is used to replace Triton X-100 in the formulation of the reagent R1, the content of Tetronic 1307 is 0.1%. The other components and concentrations of R1, as well as R2 and the test card, are the same as those in Example 1. Since Tetronic 1307 is a non-hemolytic zwitterionic surfactant, its addition to the formulation system has a certain impact on the test gradient, causing the entire gradient to decrease or even disappear.

## Claims

1. A kit for determining glycated hemoglobin, comprising:
a filtration membrane for retaining precipitated hemoglobin;
a reagent R1 comprising a boronic acid conjugate, a zinc ion, and a surfactant; and
a reagent R2 comprising a buffer solution and a surfactant,
wherein the surfactant in the reagent R1 comprises a nonionic surfactant and an anionic surfactant.

2. The kit according to claim 1, wherein in the reagent R1, the nonionic surfactant is selected from Triton X-100, and the anionic surfactant is selected from SDS.

3. The kit according to claim 1, wherein the surfactant in the reagent R1 comprises a surfactant capable of lysing an erythrocyte.

4. The kit according to claim 1, wherein the surfactant in the reagent R1 further comprises a zwitterionic surfactant.

5. The kit according to claim 4, wherein the zwitterionic surfactant in the reagent R1 is selected from one or both of Tetronic 1307 and CHAPS.

6. The kit according to claim 1, wherein the filtration membrane is disposed within a test card, the test card comprises a sample loading port, and a sample to be tested and a reagent reach the filtration membrane through the sample loading port.

7. A method for determining glycated hemoglobin, comprising:
mixing a blood sample with a reagent R1 to form a mixed sample;
passing the mixed sample through a filtration membrane capable of retaining precipitated hemoglobin;
rinsing a filtration membrane retaining the precipitated hemoglobin with a reagent R2; and
separately detecting amounts of the glycated hemoglobin and total hemoglobin, and in turn calculating a percentage of the glycated hemoglobin,
wherein
the reagent R1 comprises a boronic acid conjugate, a zinc ion, and a surfactant; and
the reagent R2 comprises a buffer solution and a surfactant,
wherein the surfactant in the reagent R1 comprises a nonionic surfactant and an anionic surfactant.

8. The method according to claim 7, wherein in the reagent R1, the nonionic surfactant is selected from Triton X-100, and the anionic surfactant is selected from SDS.

9. The method according to claim 7, wherein the surfactant in the reagent R1 comprises a surfactant capable of lysing an erythrocyte.

10. The kit according to claim 7, wherein the surfactant in the reagent R1 further comprises a zwitterionic surfactant.

11. The kit according to claim 10, wherein the zwitterionic surfactant in the reagent R1 is selected from one or both of Tetronic 1307 and CHAPS.

12. A reagent for determining glycated hemoglobin, comprising a reagent R1 and a reagent R2, wherein the reagent R1 comprises an active reactant, a metal ion, and a surfactant, and the reagent R2 is an eluent, wherein the surfactant in the reagent R1 comprises a nonionic surfactant and an anionic surfactant.

13. The reagent according to claim 12, wherein in the reagent R1, the nonionic surfactant is selected from Triton X-100, and the anionic surfactant is selected from SDS.

14. The reagent according to claim 12, wherein the surfactant in the reagent R1 comprises a surfactant capable of lysing an erythrocyte.

15. The reagent according to claim 12, wherein the surfactant in the reagent R1 further comprises a zwitterionic surfactant.

16. The reagent according to claim 15, wherein the zwitterionic surfactant in the reagent R1 is selected from one or both of Tetronic 1307 and CHAPS.

17. The reagent according to claim 12, wherein the active reactant is a boronic acid conjugate.

18. The reagent according to claim 12, wherein the metal ion in the reagent R1 comprises a zinc ion.

19. A method for preserving a reagent, wherein the reagent comprises a boronic acid conjugate, a zinc ion, and a surfactant, wherein the surfactant comprises a nonionic surfactant and an anionic surfactant.

20. The preservation method according to claim 19, wherein in the reagent R1, the nonionic surfactant is selected from Triton X-100, and the anionic surfactant is selected from SDS.

21. The preservation method according to claim 19, wherein the surfactant in the reagent R1 further comprises a zwitterionic surfactant.

22. The preservation method according to claim 21, wherein the zwitterionic surfactant in the reagent R1 is selected from one or both of Tetronic 1307 and CHAPS.
